# FASCICULE DE BREVET EUROPEEN

(11) **EP 0 802 977 B1**
(45) Date de publication et mention de la délivrance du brevet: **06.10.2004**
(21) Numéro de dépôt: 95926431.8
(22) Date de dépôt: 26.07.1995
(51) Int. Cl.: C12N 15/29, C12N 1/21, C12N 1/19, C12Q 1/68, A01H 5/00

(54) **THIOREDOXINES h DE BLE TENDRE ET DE BLE DUR ET PROTEINES PRESENTANT DES SIMILITUDES FRAGMENTS D'ADN CODANT POUR CES PROTEINES ET PROCEDES D'OBTENTION**
TIOREDIOXINE H AUS ZWIG UND HART-WEIZEN UND ÄHNLICHE PROTEINE, DIESE KODIERENDE DNA-FRAGMENTE UND VERFAHREN ZU IHRER HERSTELLUNG
HARD AND SOFT WHEAT THIOREDOXINS h, HOMOLOGOUS PROTEINS, DNA FRAGMENTS CODING FOR SAID PROTEINS AND METHODS FOR PREPARING SAME

(30) Priorité: 26.07.1994 FR 9409235
(43) Date de publication de la demande: 29.10.1997
(73) Titulaire: INSTITUT NATIONAL DE LA RECHERCHE AGRONOMIQUE (INRA), 75341 Paris Cédéx 07 (FR)
(72) Inventeur: GAUTHIER, Marie-Françoise, F-34090 Montpellier (FR); LULLIEN-PELLERIN, Valérie, F-34070 Montpellier (FR); DE LAMOTTE, Frédéric Les Portes d'Estanove, F-34070 Montpellier (FR); JOUDRIER, Philippe, F-34070 Montpellier (FR)
(74) Mandataire: Phélip, Bruno
(86) Numéro de dépôt international: PCT/FR1995/001005
(87) Numéro de publication internationale: WO 1996/003505

(56) Documents cités:
- WO-A-93/08274
- PLANT PHYSIOL., vol. 102, pages 327-328, RIVERA-MADRID, R., ET AL. 'Nucleotide sequence of a cDNA clone encoding an Arabidopsis thaliana thioredoxin h' cité dans la demande
- PLANT MOLECULAR BIOLOGY, vol. 17, pages 143-147, MARTY, I., ET AL. 'Nucleotide sequence of a cDNA encoding a tobacco thioredoxin' cité dans la demande
- EMBL SEQUENCE DATABASE REL.37. 30-11-93 ACC. NO. D21310. Rice mRNA for thioredoxin (gene name SS396), partial cds
- EMBL SEQUENCE DATABASE REL 40. 23-7-1994. ACC. NO. Z35335. A. thaliana transcribed sequence.Clone TAT6A11
- EMBL SEQUENCE DATABASE REL.38. 16-2-1994. ACC. NO. D26547. Rice gene for thioredoxin h, complete cds.
- EMBL SEQUENCE DATABASE REL.37. 26-11-1993. ACC. NO.D21836. Rice mRNA for thioredoxin h, complete cds.
- J. BIOL. CHEM. (1991), 266(3), 1692-6, GAN, ZHONG RU 'Yeast thioredoxin genes'
- J. BIOL. CHEM. (1989), 264(7), 4008-14, MULLER, ERIC G. D. ET AL 'Thioredoxin is essential for photosynthetic growth. The thioredoxin m gene of Anacystis nidulans'
- BIOCHIM. BIOPHYS. ACTA, vol. 873, no. 3, pages 415-418, VOGT, K., ET AL. 'Characterization of three different thioredoxins in wheat'
- PLANT PHYSIOLOGY, vol. 105, 1994 pages 1021-1022, JARAMILLO, J.L., ET AL. 'Cloning and sequencing of a pea cDNA fragment coding for thioredoxin m'
- PLANT MOLECULAR BIOLOGY, vol. 20, 1992 pages 301-306, AGUILAR, F., ET AL. 'Biosynthesis of active spinach-chloroplast thioredoxin f in transformed E. coli'
- PLANT PHYSIOL (BETHESDA) 99 (3). 1992. 919-924., KOBREHEL K ET AL 'SPECIFIC REDUCTION OF WHEAT STORAGE PROTEINS BY THIOREDOXIN H.' cité dans la demande

## Description

La présente invention a pour objet des thiorédoxines h de blé tendre et de blé dur, ainsi que des fragments d'ADN codant pour ces protéines.

Elle est en outre relative à des procédés d'obtention de ces protéines.

Les thiorédoxines sont des protéines de petites tailles impliquées dans divers processus biologiques et vraisemblablement présentes dans tous les organismes vivants.

Elles interviennent entre autres comme donneurs d'hydrogène pour des réductases (ribonucléotide, méthionine sulfoxyde et sulfate réductase) et comme oxydoréductases des fonctions disulfure de plusieurs protéines. Pour les propriétés générales des thiorédoxines on pourra avantageusement se référer à la revue de Pille (Annales de l'Institut Pasteur, volume 1, 34-50, 1992) ou de Holmgren (TIBS, Janvier 1981, 26-29).

Si les thiorédoxines de bactéries sont bien connues, les thiorédoxines h des organismes supérieurs, et en particulier des plantes ont été assez peu étudiées.

Ainsi, seules les thiorédoxines h de tabac (Marty et Meyer, Plant Molecular Biology, 17, 143-147, 1991; Brugidou et al., Mol Gen Genet , 238, 285-293, 1993), de riz ( séquence EMBL N° D 26547), d'Arabidopsis thaliana ( Rivera-Madrid et al., Plant Physiol, 102, 327-328,1993)et de Chlamydomonas reinhardtii (Decottignies et al. Eur. J. Biochem, 198, 505-512, 1991) ont été à ce jour séquencées.

Leur séquençage a été effectué à partir d'ADN complémentaire sélectionné dans des banques d'ADN de tabac ou d'Arabidopsis thaliana par hybridation du clone portant l'ADN complémentaire codant pour la thiorédoxine h avec une sonde correspondant à un ADN complémentaire de la thiorédoxine h1 de tabac pour Arabidopsis thaliana ( Rivera-Madrid et al. précédemment cité), c'est-à-dire une sonde hétérologue, ou après criblage par hybridation différentielle (Marty et Meyer, précédemment cités).

Zhong-Ru Gan (J. Biol. Chem, 1991, 266 (3), 1692-1696) a séquencé une thiorédoxine de levure. Des amorces correspondant à des séquences encadrant le site actif de cette thiorédoxine ont été utilisées pour amplifier un fragment de 34 paires de base. Ce fragment a alors été utilisé comme sonde dans une hybridation du type Southern pour le criblage d'une banque génomique de levure.

Muller et Buchanan (J. Biol. Chem. 1989, 264 (7), 4008-4014) ont quant à eux décrits le clonage d'un gène codant pour une thiorédoxine m, et non une thiorédoxine h. La stratégie utilisée pour le clonage consiste à faire une hybridation du type Southern du génome de la bactérie Anacystis nidulans, avec une sonde présentant des similitudes avec les sites actifs d'autres thiorédoxines m puis à cloner le fragment correspondant.

A la connaissance du demandeur, les seules séquences de thiorédoxine h de plantes qui étaient publiées, et pouvaient donc être utilisées comme sondes, étaient celles de tabac et de Chlamydomonas reinhardtii; c'est-à-dire d'une plante dicotylédone et d'une algue unicellulaire.

Ces sondes s'hybrident de manière hétérologue avec des ADN complémentaires d'autres plantes présentant une grande distance évolutive, les mono-cotylédones.

Ainsi, l'homme du métier désireux de sélectionner des clones d'ADN complémentaires dans des banques de plantes mono-cotylédones était incité à utiliser des sondes hétérologues, donc peu spécifiques, et ce d'autant plus qu'excepté le site actif, il existe peu de similarité entre les séquences de thiorédoxines h, et induisant ainsi des risques d'erreurs dans la sélection des clones empêchant toute sélection spécifique.

Or, les thiorédoxines h interviennent de manière importante chez le blé lors de la germination, et aussi en réduisant de manière spécifique les gluténines et d'autres protéines du grain de blé (Kobrehel et al, 1992, Plant Physiol., 99, 919-924). Afin d'améliorer la qualité de la farine de blé, par exemple l'état d'oxydo-réduction de certaines protéines contenues dans cette farine, on peut modifier l'activité des thiorédoxines h, au niveau génétique, en modifiant les gènes des thiorédoxines h ou en ajoutant de nouvelles copies de ces gènes ou d'ADN complémentaires correspondant à ces gènes.

Il peut être aussi envisagé de rajouter des thiorédoxines produites par des microorganismes dans des produits à usage alimentaire, ou de les utiliser pour supprimer l'effet antinutritionnel des légumineuses ou pour inactiver des toxines, par exemple de venin d'abeilles ou de serpents, Dans tous ces cas, il peut être nécessaire, voire indispensable, d'utiliser des ADN complémentaires correspondant au gène de thiorédoxine h pour produire ces protéines.

L'homme du métier se trouvait donc confronté à une absence de méthode fiable permettant la sélection dans une banque d'ADN complémentaire, de clones codant pour les thiorédoxines h.

Le demandeur s'est donc attaché à rechercher une sonde permettant de sélectionner de manière spécifique et fiable des clones de thiorédoxine h dans une banque d'ADN complémentaire.

Il a montré qu'il était possible d'effectuer une telle sélection en utilisant une sonde codant pour une séquence d'acides aminés composant le site actif des thiorédoxines.

Il a en outre montré que les thiorédoxines h de blés dur et tendre présentent d'une part une grande similitude entre elles, mais d'autre part des grandes différences de structure primaire par rapport aux autres thiorédoxines h de plantes dont les séquences sont déjà connues.

La présente invention a pour objet une thioredexine h de blé comprenant la séquence SEQ ID N° 1 suivante:

La présente invention a ainsi pour objet la thiorédoxine h de blé tendre présentant la séquence SEQ ID N°3 suivante:

Elle est en outre relative à la thiorédoxine h de blé dur présentant la séquence SEQ ID N°5 suivante:

La présente invention a en outre pour objet des fragments d'ADN codant pour une de ces protéines ou un de ces peptides et en particulier un fragment codant pour la thiorédoxine h de blé tendre comprenant la séquence SEQ ID N°2 suivante: et un fragment codant pour la thiorédoxine de blé dur comprenant la séquence SEQ ID N°4 suivante :

Elle a aussi pour objet une méthode de sélection dans une banque d'ADN complémentaire de clones codant pour une thiorédoxine h caractérisée en ce qu'on hybride lesdits clones avec une sonde présentant une similitude de séquences proche de 100% avec le site actif des thiorédoxines.

Avantageusement, une telle sonde présente la séquence suivante : (SEQ ID N° 6) dans laquelle :
X₁ représente C ou T
X₂ représente T ou A
X₃ représente A, G, C ou T
X₄ représente C ou T
X₅ représente G ou A
On remarquera, comme le montrent les comparaisons effectuées dans les exemples qui suivent , que les thiorédoxines h de blé présentent une grande différence de structure primaire par rapport aux thiorédoxines h de plantes déjà connues.

Il n'était donc en rien évident pour l'homme du métier de déduire les séquences de ces thiorédoxines h de blé des séquences d'autres thiorédoxines h divulguées dans l'état de la technique.

En outre, l'obtention d'ADN complémentaires (ADNc) pour un gène donné n'est pas, malgré les développements récents dans les techniques de biologie moléculaire, une technique de routine.

En effet, l'obtention d'un ADNc particulier nécessite la mise au point d'un procédé spécifique qui va bien au-delà d'une simple adaptation d'une technique. En particulier le choix du matériel dont sont extraits les ARN messagers est essentiel. Cette spécificité est d'autant renforcée que les ARN messagers sont en faibles quantités ce qui est le cas de la présente invention.

On notera de plus que l'utilisation d'oligonucléotides dégénérés pour cribler les ADN complémentaires n'avait jamais été mise en oeuvre dans le cas des thiorédoxines h . Il n'était en rien évident qu'une telle utilisation permette un criblage efficace.

Le blé est une graminée d'un poids économique considérable et son amélioration, ainsi que celle de ses produits en utilisant les thiorédoxines h ou des fragments d'ADN codant pour ces protéines, constituent des progrès techniques importants.

La présente invention est de plus relative à des vecteurs d'expression portant un fragment d'ADN tel que défini ci-dessus, et en particulier portant au moins une partie de la séquence SEQ ID N°2 ou de la séquence SEQ ID N°4 décrites ci-dessus.

De tels vecteurs comprennent au moins :
- une origine de réplication adaptée à l'espèce biologique, microorganisme ou autre, dans laquelle on souhaite reproduire le vecteur;
- un promoteur situé en amont du fragment d'ADN , adapté à l'espèce biologique dans laquelle on souhaite exprimer les protéines selon l'invention.

Ils peuvent aussi comprendre des séquences de régulation de l'expression du promoteur. Ce promoteur peut être soumis à régulation selon les conditions de culture des microorganismes.

De tels vecteurs peuvent être particulièrement des vecteurs de sécrétion, ou d'excrétion.

De manière avantageuse, les fragments d'ADN définis ci-dessus sont intégrés dans un plasmide, et en particulier dans le plasmide pET commercialisé par Novagen (USA).

Des vecteurs pETtrxTa et pFL61trxTa portant la séquence identifiée ci-dessus SEQ ID N°2 ont été déposés respectivement sous les numéros 1-1442 et I-1443 auprès de la Collection Nationale de Cultures de Microorganismes de l'Institut Pasteur ( CNCM).

D'autres objets de la présente invention sont des microorganismes, des cellules eucaryotes, et en particulier des cellules végétales ou animales, et des plantes transgéniques portant une des séquences définies ci-dessus. Un tel microorganisme est avantageusement une bactérie, telle que E. coli ou une corynébactérie, une levure ou un champignon filamenteux. Des cellules animales peuvent être, par exemple, des cellules d'insectes.

Les espèces biologiques portant ces fragments et/ou vecteurs sont choisies afin de permettre une expression des protéines selon l'invention.

Enfin, la présente invention est relative à un procédé de production des protéines selon l'invention, et en particulier de thiorédoxines h, comprenant les étapes suivantes :
- culture d'un microorganisme tel que défini ci-dessus, et
- isolement des protéines ou peptides selon l'invention produits par ledit microorganisme.

Le présent procédé n'est pas limité à l'obtention de dérivés de thiorédoxines h de blé. Il peut aussi être appliqué à la production de thiorédoxines h d'autres céréales telles que le maïs, l'orge, le seigle, le sorgho ou le riz, de légumineuses telles que le soja, l'haricot ou le pois ou d'oléagineux tels que le tournesol, le chanvre, le lin ou le colza, ou de dérivés de ces thiorédoxines h, à l'aide de vecteurs portant des séquences codant pour ces protéines.

Avantageusement, les microorganismes sont lysés après culture et les protéines selon l'invention sont récupérées par des méthodes connues de l'homme du métier.

L'homme du métier pourra se référer, si nécessaire, pour la préparation des protéines selon l'invention, de leurs vecteurs ou de microorganismes portant ces vecteurs, et de manière générale pour la mise en oeuvre de la présente invention au manuel suivant : Maniatis et al. Molecular cloning : A Laboratory Manual , Cold Spring Harbor Laboratory 1982 ou à une de ses récentes rééditions.

Les protéines objets de la présente invention ou pouvant être obtenues selon un procédé objet de la présente invention peuvent être utilisées dans de nombreuses applications, en particulier, comme additifs dans des produits à usage alimentaire ou non alimentaire, pour la suppression de l'effet antinutritionnel des légumineuses, pour l'inactivation de diverses toxines en particulier celles de venin d'abeilles et de serpents.

Ces applications et d'autres applications sont répertoriées dans la demande PCT/US 92/08 595 dont le contenu est intégré à la présente demande par référence.

La production de thiorédoxine h de blé dans la levure, en particulier Saccharomyces cerevisiae, permet de l'utiliser directement dans les produits alimentaires sous forme de levures enrichies en thiorédoxine h (par induction de l'expression du gène ou par accumulation de la thiorédoxine h dans la levure), sous forme lyophilisée par exemple.

Le fait d'obtenir des thiorédoxines h de blé par le procédé selon l'invention permet de les ajouter à un produit consommé par les humains tout en leur conservant leur caractère naturel.

La présente invention permet en outre d'obtenir de la thiorédoxine h de blé en quantité importante (par rapport à une purification à partir de blé) par exemple à partir de cultures de bactéries ou de levures et d'ajouter cette thiorédoxine h, après purification ou en utilisant des levures enrichies (surexprimant la thiorédoxine h), à des produits céréaliers en vue d'améliorer leur valeur d'utilisation.

Le fait de disposer des séquences codant pour les thiorédoxines h de blés dur ou tendre permet de les modifier par mutagenèse dirigée et d'obtenir des thiorédoxines h dont les propriétés sont modifiées, et en particulier dont l'activité est améliorée par rapport à celle de la thiorédoxine h isolée du blé.

La présente invention est illustrée sans pour autant être limitée par les exemples qui suivent dans lesquels:
La figure 1 illustre les différences de séquences des thiorédoxines h de blé tendre (THIOBLETA) de blé dur (THIOBLETD), de riz (THIORIZ), d'Arabidobsis (THIOARA), de thiorédoxine h2 de tabac (THIOTABAC2), de thiorédoxine h1 de tabac (THIOTABAC) et de Chlamydomonas reinhardtii (THIOCHLA).
La figure 2 illustre la construction du plasmide pETtrxTa.
Les figures 3 et 4 représentent respectivement un gel de polyacrylamide-SDS après coloration au bleu de Coomassie et un Western-blot effectué avec un anticorps dirigé contre la thiorédoxine h de blé de :
   1. lysat de bactéries avant induction,
   2. culot des protéines insolubles du lysat après 3h d'induction,
   3. culot après chauffage des protéines solubles du lysat,
   4.surnageant après chauffage des protéines solubles du lysat,
   5. comme 2 après 6h d'induction,
   6. comme 3 après 6h d'induction,
   7. comme 4 après 6h d'induction.

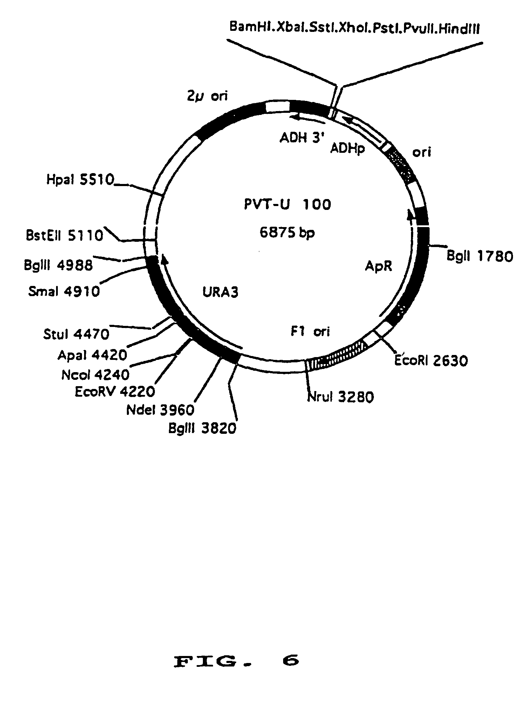

Les figures 5 et 6 représentent schématiquement les plasmides pFL61 et pVT-U 100.

### EXEMPLE 1: Obtention de clones de thiorédoxine h de blé tendre

### 1°) Construction de la banque d'ADN complémentaire (ADNc).

L'extraction des ARN totaux de graines et la sélection des ARN poly (A)⁺ ont été effectuées comme décrit par Gautier et al. ( Plant Mol Biol., 14, 313-322, 1990).

5µg d'ARN poly (A)⁺ issus de graines de Triticum aestivum L., variété capitole en cours de maturation (23 jours après floraison ) ont été utilisés pour construire une banque d'ADN complémentaire, en utilisant le Système Superscript Plasmid commercialisé par BRL.

Les ADN complémentaires présentant une taille supérieure à 500 pb sont ligués au plasmide pSPORT1 commercialisé par BRL coupé par les enzymes NotI-SalI, qui est utilisé pour transformer des cellules d'Escherichia coli DH5α.

2.10⁵ bactéries recombinantes sont obtenues avant amplification de la banque. Environ 3000 recombinants sont étalés et les colonies sont transférées sur une membrane Hybond C (Amersham) selon les instructions du fabricant.

### 2°) Isolement d'un clone codant pour une thiorédoxine h de blé tendre.

La banque d'ADN complémentaire obtenue en 1°) est criblée à l'aide d'un mélange d'oligonucléotides de synthèse présentant la séquence ID N° 6 suivante : TGGTGX₁GGX₂CCX₃TGX₄AAX₅ATG
dans laquelle :
X₁ représente C ou T
X₂ représente T ou A
X₃ représente A, G, C ou T
X₄ représente C ou T
X₅ représente G ou A

Un mélange contenant ces oligonucléotides synthétiques marqués à leurs extrémités 5' par du gamma-³²P ATP à l'aide de la polynucléotide kinase T4 a été utilisé.

Les filtres ont été préhybridés (16 heures , 37°C) et hybridés (4 heures, 37°C) dans une solution comprenant 15% (v/v) de formamide désionisé, SSPE 2 X, solution de Denhardt 5 X, SDS 1 % (poids/volume) et de l'ADN de sperme de saumon dénaturé (200 µg/ml).

Les filtres hybridés sont lavés deux fois dans du SSPE 2 X et du SDS 0,1 % ( poids/volume) durant 10 minutes à température ambiante; puis deux fois dans du SSPE 0,25 X, et du SDS 0,1 % (poids/volume) durant 30 minutes à 37°C puis une fois dans du SSPE 0,25 X durant 10 minutes à 37°C.

Ils sont ensuite exposés à des films sensibles aux rayons X ( Fuji ) à -70°C avec deux écrans intensifiants.

Un clone, appelé pTaM1338, est isolé et sa séquence est déterminée sur les deux brins en utilisant la trousse de séquençage Taq Dye Deoxy Terminator Cycle Sequencing kit commercialisé par Applied Biosystems et le séquenceur 370 DNA automatique commercialisé par Applied Biosystems.

La séquence de l'ADN complémentaire du clone pTaM1338 est la suivante :
(SEQ ID N°7)

L'extrémité 5' de cette séquence comprend une séquence de 63 paires de bases (pb) non codante, suivie d'une phase de lecture ouverte de 381 pb, puis d'une séquence non codante de 215 pb, à l'extrémité 3'.

La phase de lecture ouverte code pour une protéine de 127 acides aminés de séquence SEQ ID N°2.

La masse théorique de la protéine codée par cette phase de lecture ouverte est de 13524D.

### EXEMPLE 2:

### Obtention de clone de thiorédoxine h de blé dur.

### 1) Construction de la banque d'ADN complémentaire de blé dur.

La banque est obtenue de manière similaire à celle de l'exemple 1 à l'exception du matériel végétal utilisé qui est Triticum durum Desf. Variété Agathé. Les ARN totaux sont isolés de grains 22 jours après floraison.

Les ARN messagers isolés par chromatographie d'affinité sur oligo dT cellulose sont clonés dans le plasmide pUC118 dans le site de clonage PstI.

La souche d'Escherichia coli JM109 est transformée avec les plasmides obtenus.

La méthode de fabrication de cette banque d'ADN complémentaire est mise en oeuvre de la manière décrite par Gautier et al. (Plant Molecular Biology, 14, 313-322, 1990) dont la publication est incluse par référence à la présente demande.

### 2. Isolement d'un clone codant pour une thiorédoxine h de blé dur.

Des clones sont criblés comme indiqué dans l'exemple 1 par le même mélange d'oligonucléotides de synthèse ( SEQ ID N° 6).

Un clone, dénommé pTd14132 est isolé et sa séquence est déterminée comme indiqué dans l'exemple 1.

Ce clone comprend la séquence d'ADN complémentaire de blé dur suivante :
SEQ ID N°8

L'extrémité 5' de cette séquence comprend une partie non codante de 50 bp, puis une phase de lecture ouverte de 390 pb puis une partie non codante de 190 pb à son extrémité 3'.

La phase de lecture ouverte correspond à une protéine de 130 acides aminés, ayant une masse moléculaire théorique de 13750D.

### EXEMPLE 3:

### Comparaison des structures primaires des thiorédoxines h de blés dur et tendre et des autres thiorédoxines h divulguées dans l'état de la technique.

Les structures primaires des deux protéines correspondant aux clones pTaM1338 et pTd14132 ont été comparées entre elles et aux structures primaires de thiorédoxines h de riz ( THIORIZ), de thiorédoxine h d'Arabidopsis (THIOARA) , de thiorédoxine h2 de tabac (THIOTABAC 2), de thiorédoxine h1 de tabac (THIOTABAC) et de thiorédoxine h de Chlamydomonas reinhardtii (THIOCHLA).

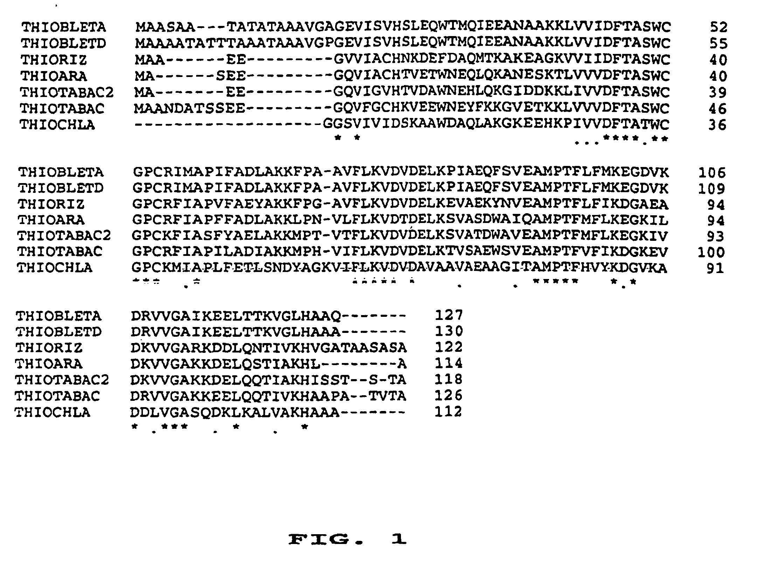

Les résultats de ces comparaisons sont repris dans la figure 1.

Dans cette figure les acides aminés sont représentés par le code à une lettre et (*) représente une position d'acide aminé identique dans les sept protéines, tandis que (.) représente une position d'acide aminé similaire.

Sur une longueur totale de 138 acides aminés, on observe une conservation à l'identique pour 31 acides aminés (22,5 %) et une similarité pour 42 acides aminés ( 30,4 %).

Il ressort donc clairement de cette figure que les thiorédoxines h de blés montrent une faible identité de séquence avec les autres thiorédoxines h de végétaux déjà séquencés.

De manière surprenante, l'identité de séquence entre d'une part la thiorédoxine h de riz et d'autre part les thiorédoxines h de blé tendre et de blé dur n'est que de respectivement 54,9% et 55,7 %, alors que ces plantes sont toutes trois des graminées.

### EXEMPLE 4:

### Production de thiorédoxine h par des bactéries.

### 1. Sous-clonage de la séquence codant pour la thiorédoxine h de blé tendre dans un vecteur d'expression d'E.coli:

Le DNA plasmidique pTAM1338 contenant la séquence d'ADNc codant pour la thiorédoxine h de blé tendre (Triticum aestivum) a été modifié par mutagénèse dirigée pour introduire les sites de restriction NdeI et BamHI respectivement en 5' et 3' de la séquence codant pour la protéine.

Ces sites de restriction ont ensuite servi à introduire la séquence codant pour la thiorédoxine h de blé tendre (Triticum aestivum) dans le vecteur d'expression pET3b commercialisé par Novagen (USA) et décrit par Rosenberg et al., (Gene, 56, 125-135, 1987) digéré par les mêmes enzymes.

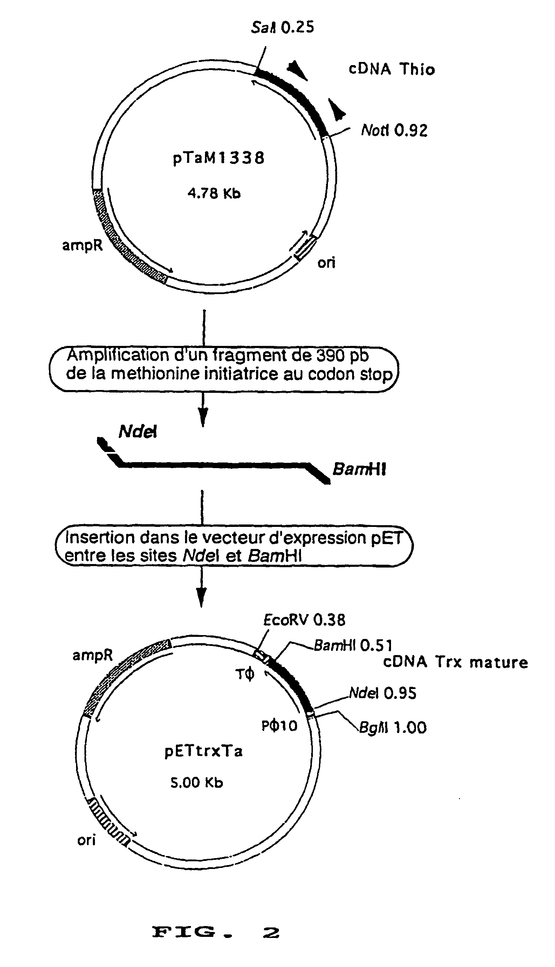

La figure 2 illustre cette construction.

Le vecteur pET3b est une molécule d'ADN circulaire dérivé de pBR322; il contient les éléments suivants :
- le promoteur du gène 10 reconnu par l'ARN polymérase T7 (appelé PO10) contenu entre les sites de restriction BglII et XbaI,
- la séquence Shine-Dalgarno du gène 10,
- un codon d'initiation ATG contenu dans le site unique de restriction NdeI en 5' des premiers codons du gène 10,
- un site de restriction unique BamHI qui permet de cloner une séquence d'un gène étranger dans le vecteur d'expression,
- le terminateur de transcription qui suit normalement le gène 10(TO).

Ce vecteur possède le replicon pMB1 (ori) et contient le gène bla qui code pour la résistance à l'ampiciline (ampR).

La séquence codant pour la thiorédoxine h de blé tendre incluse entre les sites de restriction NdeI et BamHI qui ont été créés par mutagénèse dirigée est introduite dans le vecteur d'expression digéré par les mêmes enzymes.

Le vecteur résultant pETtrxTa est utilisé pour transformer des souches d'E. coli.

Les méthodes conventionnelles de clonage ont été utilisées. Elles sont décrites par Maniatis et al. (1982). Molecular Cloning: A Laboratory Manual, 2nd Ed., Cold Spring Harbor Laboratory, New York.

Le plasmide pETtrxTa résultant de la construction a été séquencé comme décrit par Sanger et al. (1977, Proc. Natl. Acad. Sci. USA 74, 5463-5467), dans le but de vérifier qu'aucune mutation n'a été introduite dans la séquence de la thiorédoxine h au cours de l'amplification ou du clonage.

La séquence codant pour la thiorédoxine h peut aussi être introduite dans le vecteur d'expression après modification, par mutagénèse dirigée, d'un ou plusieurs acides aminés de la protéine dans le but de changer ses propriétés. Les méthodes conventionnelles de mutagénèse dirigée sont décrites par Maniatis et al. (1982, précédemment cité).

### 2. Obtention de bactéries produisant de la thiorédoxine h:

Le vecteur pETtrxTa qui contient la séquence codant pour la thiorédoxine h de blé sous contrôle du promoteur reconnu par l'ARN polymérase du bactériophage T7 est utilisé pour transformer des souches d'E. coli (Hanahan et al., 1985, Technique for transformation of E. coli in "DNA Cloning: A practical Approach "(Glover , D.M.Ed. Vol.1, pp 109-135, IRL Press, Oxford), capables de synthétiser l'ARN polymérase T7. De telles souches sont commercialisées par Novagen (USA) et décrites par Studier et al., (1990, Methods Enzymol. 185, 60-89). Elles peuvent être:
- BL21 (DE3): ompT hsdS gal ( lambda cIts857 ind1 Sam7 nin5 lacUV5-T7 gene1),
- BL21 (DE3)pLysE: même génotype que BL21 (DE3) excepté le plasmide pLysE qui dérive du plasmide pACYC184 (Chang et al., 1978 , J.Bacteriol. 134-1141) et contient le gène codant pour le lysozyme T7 ainsi que le gène de résistance au chloramphénicol. Le gène codant pour le lysozyme est exprimé à partir du promoteur tet de pACYC184 ce qui signifie que les bactéries qui portent ce plasmide accumulent un taux important de lysozyme.
- BL21 (DE3)pLysS: même génotype que BL21 (DE3)pLysE mais le gène codant pour le lysozyme est inséré dans l'orientation opposée. En conséquence, les bactéries qui portent ce plasmide accumulent une quantité beaucoup plus faible de lysozyme.

Les bactéries transformées sont multipliées dans le milieu de Luria-Bertani avec les antibiotiques nécessaires, à 30°C.

### 3. Analyse de l'expression de la thiorédoxine h dans les bactéries.

Les bactéries contenant le vecteur pETtrxTa sont cultivées jusqu'à une densité optique comprise entre 0,3 et 0,6 à 600 nm, (une fraction aliquote avant induction est conservée pour analyse). L'inducteur de l'expression de l'ARN polymérase T7 (IPTG 0.1 mM) est alors ajouté au milieu de culture pour permettre l'expression de la thiorédoxine h et les bactéries sont collectées par centrifugation après 3 ou 6 h d'induction.

Les bactéries induites sont lysées par les méthodes conventionnelles et le lysat contenant les protéines totales est centrifugé pour séparer la fraction "protéines insolubles" (culot) de celle des "protéines solubles" ( surnageant).

Le surnageant qui contient l'activité thiorédoxine h, identifiée par dosage de la réduction de la malate déshydrogénase comparable au témoin extrait de blé, est chauffé à 60°C (5 min.) et centrifugé pour séparer la fraction des protéines thermostables (surnageant) des autres protéines.

Les échantillons des différentes fractions sont traités avec le tampon de charge de Laemlli (Laemlli, 1970, Nature, 227, 680-685), chauffé 5 à 10 minutes dans un bain marie bouillant et analysé par gel de sodium dodécyl sulfate-polyacrylamide.

Une protéine de la taille attendue pour une thiorédoxine h de blé est présente dans le lysat des protéines totales de bactéries induites et reste soluble même après chauffage à 60°C; le même gel est transféré sur une membrane de nitrocellulose (Towbin et al., 1979, Proc. Natl. Acad. Sci. USA 76, 435P-4354), et incubé avec un anticorps dirigé contre la thiorédoxine h de blé. La protéine, de taille attendue, synthétisée dans le cytoplasme bactérien après induction, réagit avec l'anticorps.

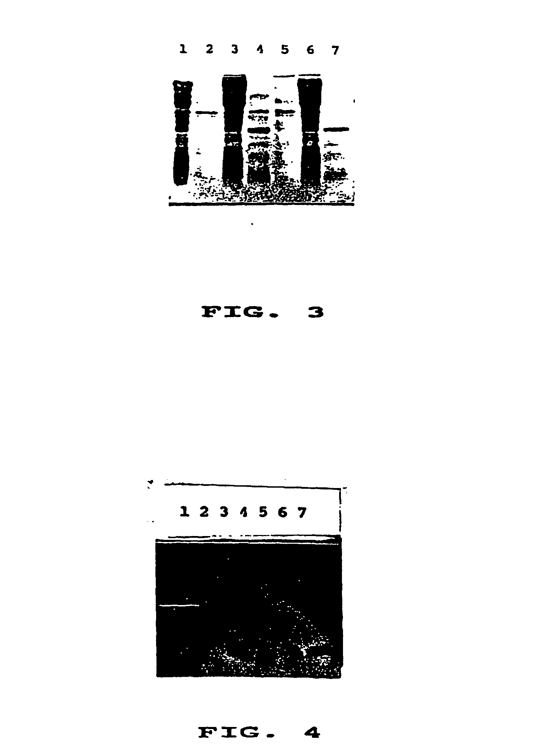

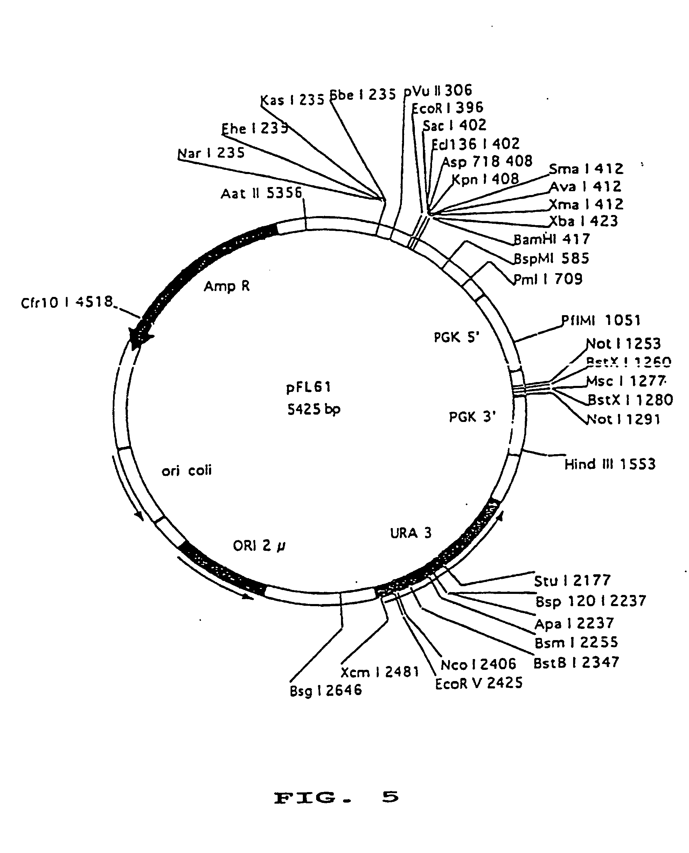

Les figures 3 et 4 représentent respectivement un gel de polyacrylamide-SDS après coloration au bleu de Coomassie et un Western-Blot effectué avec un anticorps dirigé contre la thiorédoxine h de blé, de :
1. lysat de bactéries avant induction,
2. culot des protéines insolubles du lysat après 3h d'induction,
3. culot après chauffage des protéines solubles du lysat,
4.surnageant après chauffage des protéines solubles du lysat,
5. comme 2 après 6h d'induction,
6. comme 3 après 6h d'induction,
7. comme 4 après 6h d'induction.

### 4. Purification de la thiorédoxine h de blé.

Les conditions de purification utilisées suivent essentiellement le protocole décrit par de Lamotte-Guéry et al., ((1991) Eur. J. Biochem. 196, 287-294). Les bactéries sont récoltées après induction de 4h selon les conditions décrites plus haut et resuspendues dans un tampon 30 mM Tris/HCl pH 7,9 et 1 mM EDTA (tampon A).

Après un cycle de congélation (-20°C)/décongélation les cellules sont lysées avec une presse de French et le lysat ainsi obtenu est centrifugé à 4°C, 30 minutes à 50 000 g pour récupérer la fraction surnageante qui est ensuite chauffée à 60°C, 5 minutes.

Les protéines dénaturées par le traitement à chaud sont centrifugées comme précédemment. Le surnageant contient principalement la thiorédoxine h. Elle peut être purifiée par précipitation au sulfate d'ammonium (35-80 %) suivie d'une chromatographie d'exclusion (Sephadex G-50) et d'une chromatographie échangeuse d'ions (Q-Hyper D).

Cette dernière chromatographie est réalisée avec un gradient de O à 200 mM NaCl, la thiorédoxine h de blé produite dans E.coli est éluée à une concentration de 90 mM NaCl. La mesure de l'activité de la thiorédoxine h (mesure de l'activation de la malate déshydrogénase à NADP selon Jacquot et al.((1981), Plant Physiol., 68, 300-304) à chaque étape aide à suivre la purification.

### EXEMPLE 5 : Production de thiorédoxine h par des levures.

### 1. Construction de pFL61trxTa:

Le fragment correspondant à la séquence codante de pTaM1388 est amplifié en utilisant deux oligonucléotides de synthèse s'hybridant aux régions 15-34 et 482-502 et un site de restriction NotI est ajouté à chaque extrémité.

Le fragment résultant est inséré dans le vecteur pFL61 représenté sur la figure 5 ( Lacroute, (1992) Plant J.2 (3), 417-422) préalablement linéarisé par NotI.

Le sens d'insertion et la séquence sont contrôlés. Le vecteur résultant est appelé pFL61trxTa.

### 2. Construction de pVTUtrxTa:

La séquence de l'ADNc codant pour la thiorédoxine h de blé tendre issue de pTaM1338 est isolée après digestion par BamHI et NdeI du plasmide pETtrxTa (plasmide pET portant la séquence codante de pTaM1338) puis insérée dans le vecteur pVTUtrxTa représenté sur la figure 6, (Vernet et al. (1987) Gene 52, 225-233 ) au niveau du site de clonage Pvu II. Le vecteur résultant est appelé pVTUtrxTa.

### 3. Conditions de purification:

Les levures (souche OL1 et YPH 250) sont transformées par pVTUtrxTa et sont cultivées en milieu liquide à 30°C et en conditions sélectives, permettant le maintien des plasmides dans les cellules jusqu'à une absorbance à 550 nm de 1, puis sont transférées en milieu riche pendant 16 heures.

Ceci permet d'augmenter la biomasse et le faible nombre de divisions ayant lieu pendant cette durée de temps limite les effets de perte de plasmide. Les cellules sont ensuite cassées par passage dans un broyeur à billes ou par incubation dans de l'ammoniaque. Les conditions de purification de la protéine recombinante à partir du lysat cellulaire sont celles décrites par de Lamotte et al.( (1991). Eur. J. Biochem. 196, 287-294).

Les deux souches de levures transformées produisent des thiorédoxines h décelables par immunoempreintes.

La souche YPH252 déposée à l'ATCC peut aussi être utilisée.

## Revendications

1. Thioredoxine h de blé comprenant la séquence SEQ ID N° 1 suivante :

2. Thiorédoxine h de blé tendre selon la revendication 1 présentant la séquence suivante: SEQ ID N°3

3. Thiorédoxine h de blé dur selon la revendication 1 présentant la séquence suivante: SEQ ID N°5

4. Fragment d'ADN codant pour la thiorédoxine h de blé tendre selon la revendication 2, **caractérisé en ce qu'**il comprend la séquence suivante : SEQ ID N°2

5. Fragment d'ADN codant pour la thiorédoxine h de blé dur selon la revendication 3, **caractérisé en ce qu'**il comprend la séquence suivante : SEQ ID N° 4

6. Vecteur nucléotidique portant un fragment d'ADN selon l'une des revendications 4 et 5.

7. Vecteur appelé pETtrxTa selon la revendication 6 portant la séquence SEQ ID N°2, déposé auprès de la CNCM sous le n°I-1442.

8. Vecteur appelé pFL61trxTa selon la revendication 6 portant la séquence SEQ ID N°2, déposé auprès de la CNCM sous le n° I-1443.

9. Microorganisme portant un vecteur selon l'une des revendications 6 à 8

10. Microorganisme selon la revendication 9 **caractérisé en ce qu'**il est une bactérie ou une levure.

11. Méthode de sélection dans une banque d'ADN complémentaires de clones codant pour une thiorédoxine h **caractérisée en ce que** l'on hybride lesdits clones avec une sonde présentant une similitude de séquences proche de 100 % avec le site actif des thiorédoxines.

12. Méthode selon la revendication 11 **caractérisée en ce que** ladite sonde présente la séquence suivante : SEQ ID N°6 dans laquelle :
X₁ représente C ou T
X₂ représente T ou A
X₃ représente A, G, C ou T
X₄ représente C ou T
X₅ représente G ou A

13. Procédé de production de protéines et de peptides selon l'une des revendications 1 à 3, et en particulier de thiorédoxines h comprenant les étapes suivantes :
- culture d'un microorganisme selon l'une des revendications 9 et 10, et
- isolement des protéines ou peptides selon l'une des revendications 1 à 4 produits par ledit microorganisme.

14. Procédé selon la revendication 13
**caractérisé en ce que** les microorganismes sont lysés après culture.

15. Plante transgénique **caractérisée en ce qu'**elle porte un fragment d'ADN selon l'une des revendications 4 ou 5.

## Claims

1. A wheat thioredoxin h having the following sequence SEQ ID N° 1

2. A soft wheat thioredoxin h according to claim 1, having the following sequence: SEQ ID N°3

3. A hard wheat thioredoxin h according to claim 1, having the following sequence: SEQ ID N°5

4. A DNA fragment, encoding for soft wheat thioredoxin h, according to claim 2, **characterised in that** it comprises the following sequence: SEQ ID N°2

5. A DNA fragment; encoding for hard wheat thioredoxin h, according to claim 3, **characterised in that** it comprises the following sequence: SEQ ID N°4

6. A nucleotide vector carrying a DNA fragment according to one of claims 4 and 5.

7. A vector called pETtrxTa according to claim 6, having the sequence SEQ ID N°2 filed at CNCM under the number I-1442.

8. A vector called pFL61trxTa according to claim 6, having the sequence SEQ ID N°2 filed at CNCM under the number I-1443.

9. A micro-organism carrying a vector according to one of claims 6 to 8.

10. A micro-organism according to claim 9, **characterised in that** it is a bacteria or a yeast.

11. A method for selecting in an encoding clone complementary DNA bank for a thioredoxin h **characterised in that** said clones are hybridized with a probe having a sequence similarity close to 100% with the active site of the thioredoxins.

12. A method according to claim 11 **characterised in that** said probe has the following sequence: SEQ ID N°6 Wherein:
X₁ represents C or T
X₂ represents T or A
X₃ represents A, G, C or T
X₄ represents C or T
X₅ represents G or A.

13. A method for producing proteins and peptides according to one of claims 1 to 3, and in particular thioredoxins h comprising the following steps:
- growth of a micro-organism according to one of claims 9 and 10,
- isolation of the proteins or peptides according to one of claims 1 to 4 produced by said micro-organism.

14. A method according to claim 13, **characterised in that** the microorganisms are lysed after growth.

15. A transgenic plant, **characterised in that** it carries a DNA fragment according to one of claims 4 or 5.

## Patentansprüche

1. Weizen-Thioredoxin h, umfassend die folgende Sequenz SEQ ID Nr. 1:

2. Weichweizen-Thioredoxin h gemäß Anspruch 1, das die folgende Sequenz SEQ ID Nr. 3 aufweist:

3. Hartweizen-Thioredoxin h gemäß Anspruch 1, das die folgende Sequenz SEQ ID Nr. 5 aufweist:

4. DNA-Fragment, das für das Weichweizen-Thioredoxin h gemäß Anspruch 2 codiert, **dadurch gekennzeichnet, dass** es die folgende Sequenz SEQ ID Nr. 2 umfasst:

5. DNA-Fragment, das für das Hartweizen-Thioredoxin h gemäß Anspruch 3 codiert, **dadurch gekennzeichnet, dass** es die folgende Sequenz SEQ ID Nr. 4 umfasst:

6. Nucleotidvektor, der ein DNA-Fragment gemäß einem der Ansprüche 4 und 5 trägt.

7. pETtrxTa genannter Vektor gemäß Anspruch 6, der die Sequenz SEQ ID Nr. 2 trägt und bei der CNCM unter der Nr. I-1442 hinterlegt ist.

8. pFL61trxTa genannter Vektor gemäß Anspruch 6, der die Sequenz SEQ ID Nr. 2 trägt und bei der CNCM unter der Nr. I-1443 hinterlegt ist.

9. Mikroorganismus, der einen Vektor gemäß einem der Ansprüche 6 bis 8 trägt.

10. Mikroorganismus gemäß Anspruch 9, **dadurch gekennzeichnet, dass** es sich um ein Bakterium oder eine Hefe handelt.

11. Verfahren zur Auswahl von Klonen, die für ein Thioredoxin h codieren, aus einer cDNA-Bank, **dadurch gekennzeichnet, dass** die Klone mit einer Sonde hybridisiert werden, die eine Sequenzhomologie mit dem aktiven Zentrum der Thioredoxine in der Nähe von 100% hat.

12. Verfahren gemäß Anspruch 11, **dadurch gekennzeichnet, dass** die Sonde die folgende Sequenz aufweist: SEQ ID Nr. 6 wobei
X₁ C oder T darstellt;
X₂ T oder A darstellt;
X₃ A, G, C oder T darstellt;
X₄ C oder T darstellt;
X₅ G oder A darstellt.

13. Verfahren zur Herstellung von Proteinen und Peptiden gemäß einem der Ansprüche 1 bis 3 und insbesondere von Thioredoxinen h, umfassend die folgenden Schritte:
- Kultivieren eines Mikroorganismus gemäß einem der Ansprüche 9 und 10; und
- Isolieren der Proteine oder Peptide gemäß einem der Ansprüche 1 bis 4, die von diesem Mikroorganismus erzeugt werden.

14. Verfahren gemäß Anspruch 13, **dadurch gekennzeichnet, dass** die Mikroorganismen nach der Kultur lysiert werden.

15. Transgene Pflanze, **dadurch gekennzeichnet, dass** sie ein DNA-Fragment gemäß einem der Ansprüche 4 oder 5 trägt.


### LISTE DE SEQUENCES

(1) INFORMATION GENERALE:
   (i) DEPOSANT:
      (A) NOM: Institut National de la Recherche Agronomique INRA
      (B) RUE: 147 rue de l'université
      (C) VILLE: Paris
      (E) PAYS: France
      (F) CODE POSTAL: 75348
   (ii) TITRE DE L'INVENTION: Thiorédoxines h de blé tendre et de blé dur et protéines présentant des similitudes; fragmente d'ADN codant pour ces protéines et procédés d'obtention
   (iii) NOMBRE DE SEQUENCES: 8
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 109 acides aminés
      (B) TYPE: acide aminé
      (D) CONFIGURATION: linéaire
   (ii) TYPE DE MOLECULE: peptide
   (iii) HYPOTHETIQUE: NON
   (vi) ORIGINE:
      (A) ORGANISME: Triticum aestivum
   (xi) DESCRIPTION DE LA SEQUENCE: SEQ ID NO: 1:
   Gly Glu Val Ile Ser Val His Ser Leu Glu Gln Trp Thr Met Gln Ile
   1               5                   10                  15
   Glu Glu Ala Asn Ala Ala Lys Lys Leu Val Val Ile Asp Phe Thr Ala
               20                  25                  30
   Ser Trp Cys Gly Pro Cys Arg Ile Met Ala Pro Ile Phe Ala Asp Leu
           35                  40                  45
   Ala Lys Lys Phe Pro Ala Ala Val Phe Leu Lys Val Asp Val Asp Glu
       50                  55                  60
   Leu Lys Pro Ile Ala Glu Gln Phe Ser Val Glu Ala Met Pro Thr Phe
   65                  70                  75                  80
   Leu Phe Met Lys Glu Gly Asp Val Lys Asp Arg Val Val Gly Ala Ile
                   85                  90                  95
   Lys Glu Glu Leu Thr Thr Lys Val Gly Leu His Ala Ala
               100                 105
(2) INFORMATION POUR LA SEQ ID NO: 2:
   (i) CARACTERISTIQUES DE LA SEQUENCE:
      (A) LONGUEUR: 384 paires de bases
      (B) TYPE: acide nucléique
      (C) NOMBRE DE BRINS: deux
      (D) CONFIGURATION: linéaire
   (ii) TYPE DE MOLECULE: ADNc pour ARNm
   (iii) HYPOTHETIQUE: NON
   (vi) ORIGINE:
      (A) ORGANISME: Triticum aestivum
   (ix) CARACTERISTIQUE ADDITIONELLE:
      (A) NOM/CLE: CDS
      (B) EMPLACEMENT: 1..381
   (xi) DESCRIPTION DE LA SEQUENCE: SEQ ID NO: 2:
   ATG GCG GCG TCG GCG GCG ACG GCG ACG GCG ACG GCG GCG GCG GTA GGG        48
   Met Ala Ala Ser Ala Ala Thr Ala Thr Ala Thr Ala Ala Ala Val Gly
     1               5                  10                  15
   GCG GGG GAG GTG ATC TCC GTC CAC AGC CTG GAG CAG TGG ACC ATG CAG        96
   Ala Gly Glu Val Ile Ser Val His Ser Leu Glu Gln Trp Thr Met Gln
                20                  25                  30
   ATC GAG GAG GCC AAC GCC GCC AAG AAG CTG GTG GTG ATT GAC TTC ACT       144
   Ile Glu Glu Ala Asn Ala Ala Lys Lys Leu Val Val Ile Asp Phe Thr
            35                  40                  45
   GCA TCA TGG TGC GGA CCA TGC CGC ATT ATG GCT CCA ATT TTC GCT GAT       192
   Ala Ser Trp Cys Gly Pro Cys Arg Ile Met Ala Pro Ile Phe Ala Asp
        50                  55                  60
   CTC GCC AAG AAG TTC CCA GCT GCT GTT TTC CTC AAG GTC GAC GTT GAT       240
   Leu Ala Lys Lys Phe Pro Ala Ala Val Phe Leu Lys Val Asp Val Asp
    65                  70                  75                  80
   GAA CTG AAG CCC ATT GCT GAG CAA TTC AGC GTG GAG GCC ATG CCA ACC       288
   Glu Leu Lys Pro Ile Ala Glu Gln Phe Ser Val Glu Ala Met Pro Thr
                    85                  90                  95
   TTC CTG TTC ATG AAG GAA GGA GAT GTC AAG GAC AGG GTT GTC GGA GCT       336
   Phe Leu Phe Met Lys Glu Gly Asp Val Lys Asp Arg Val Val Gly Ala
               100                 105                 110
   ATC AAG GAG GAA CTG ACG ACC AAG GTT GGG CTA CAC GCG GCC CAG           381
   Ile Lys Glu Glu Leu Thr Thr Lys Val Gly Leu His Ala Ala Gln
           115                 120                 125
   TAA                                                                   384
(2) INFORMATION POUR LA SEQ ID NO: 3:
   (i) CARACTERISTIQUES DE LA SEQUENCE:
      (A) LONGUEUR: 127 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 3:
   Met Ala Ala Ser Ala Ala Thr Ala Thr Ala Thr Ala Ala Ala Val Gly
    1               5                  10                  15
   Ala Gly Glu Val Ile Ser Val His Ser Leu Glu Gln Trp Thr Met Gln
               20                  25                  30
   Ile Glu Glu Ala Asn Ala Ala Lys Lys Leu Val Val Ile Asp Phe Thr
           35                  40                  45
   Ala Ser Trp Cys Gly Pro Cys Arg Ile Met Ala Pro Ile Phe Ala Asp
       50                  55                  60
   Leu Ala Lys Lys Phe Pro Ala Ala Val Phe Leu Lys Val Asp Val Asp
   65                  70                  75                  80
   Glu Leu Lys Pro Ile Ala Glu Gln Phe Ser Val Glu Ala Met Pro Thr
                   85                  90                  95
   Phe Leu Phe Met Lys Glu Gly Asp Val Lys Asp Arg Val Val Gly Ala
               100                 105                 110
   Ile Lys Glu Glu Leu Thr Thr Lys Val Gly Leu His Ala Ala Gln
           115                 120                 125
(2) INFORMATION POUR LA SEQ ID NO: 4:
   (i) CARACTERISTIQUES DE LA SEQUENCE:
      (A) LONGUEUR: 393 paires de bases
      (B) TYPE: acide nucléique
      (C) NOMBRE DE BRINS: deux
      (D) CONFIGURATION: linéaire
   (ii) TYPE DE MOLECULE: ADNc pour ARNm
   (iii) HYPOTHETIQUE: NON
   (vi) ORIGINE:
      (A) ORGANISME: Triticum durum
   (ix) CARACTERISTIQUE ADDITIONELLE:
      (A) NOM/CLE: CDS
      (B) EMPLACEMENT: 1..390
   (xi) DESCRIPTION DE LA SEQUENCE: SEQ ID NO: 4:
   ATG GCG GCG GCG GCG ACG GCG ACG ACT ACA GCG GCG GCG ACG GCG GCG    48
   Met Ala Ala Ala Ala Thr Ala Thr Thr Thr Ala Ala Ala Thr Ala Ala
     1               5                  10                  15
   GCG GTG GGG CCG GGG GAG GTG ATC TCC GTC CAC AGC CTG GAG CAG TGG    96
   Ala Val Gly Pro Gly Glu Val Ile Ser Val His Ser Leu Glu Gln Trp
                20                  25                  30
   ACC ATG CAG ATC GAG GAG GCC AAC GCC GCC AAG AAG CTG GTG GTG ATT   144
   Thr Met Gln Ile Glu Glu Ala Asn Ala Ala Lys Lys Leu Val Val Ile
            35                  40                  45
   GAC TTC ACT GCA TCA TGG TGC GGA CCA TGC CGC ATC ATG GCT CCA ATT   192
   Asp Phe Thr Ala Ser Trp Cys Gly Pro Cys Arg Ile Met Ala Pro Ile
        50                  55                  60
   TTT GCT GAT CTC GCC AAG AAG TTC CCA GCT GCT GTT TTC CTC AAG GTC   240
   Phe Ala Asp Leu Ala Lys Lys Phe Pro Ala Ala Val Phe Leu Lys Val
    65                  70                  75                  80
   GAC GTT GAT GAA CTG AAG CCC ATT GCT GAG CAA TTC AGC GTC GAG GCC   288
   Asp Val Asp Glu Leu Lys Pro Ile Ala Glu Gln Phe Ser Val Glu Ala
                    85                  90                  95
   ATG CCA ACC TTC CTG TTC ATG AAG GAA GGA GAC GTC AAG GAC AGG GTT   336
   Met Pro Thr Phe Leu Phe Met Lys Glu Gly Asp Val Lys Asp Arg Val
               100                 105                 110
   GTC GGA GCT ATC AAG GAG GAG CTG ACG ACC AAG GTT GGG CTC CAC GCG   384
   Val Gly Ala Ile Lys Glu Glu Leu Thr Thr Lys Val Gly Leu His Ala
           115                 120                 125
   GCT GCC TAG                                                       393
   Ala Ala
       130
(2) INFORMATION POUR LA SEQ ID NO: 5:
   (i) CARACTERISTIQUES DE LA SEQUENCE:
      (A) LONGUEUR: 130 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 5:
   Met Ala Ala Ala Ala Thr Ala Thr Thr Thr Ala Ala Ala Thr Ala Ala
   1 5 10 15
   Ala Val Gly Pro Gly Glu Val Ile Ser Val His Ser Leu Glu Gln Trp
   20 25 30
   Thr Met Gln Ile Glu Glu Ala Asn Ala Ala Lys Lys Leu Val Val Ile
   35 40 45
   Asp Phe Thr Ala Ser Trp Cys Gly Pro Cys Arg Ile Met Ala Pro Ile
   50 55 60
   Phe Ala Asp Leu Ala Lys Lys Phe Pro Ala Ala Val Phe Leu Lys Val
   65 70 75 80
   Asp Val Asp Glu Leu Lys Pro Ile Ala Glu Gln Phe Ser Val Glu Ala
   85 90 95
   Met Pro Thr Phe Leu Phe Met Lys Glu Gly Asp Val Lys Asp Arg Val
   100 105 110
   Val Gly Ala Ile Lys Glu Glu Leu Thr Thr Lys Val Gly Leu His Ala
   115 120 125
   Ala Ala
   130
(2) INFORMATION POUR LA SEQ ID NO: 6:
   (i) CARACTERISTIQUES DE LA SEQUENCE:
      (A) LONGUEUR: 21 bases
      (B) TYPE: acide nucléique
      (C) NOMBRE DE BRINS: un
      (D) CONFIGURATION: linéaire
   (ii) TYPE DE MOLECULE: ADN
   (iii) HYPOTHETIQUE: NON
   (v) TYPE DU FRAGMENT: interne
   (ix) CARACTERISTIQUE ADDITIONELLE:
      (A) NOM/CLE: variation
      (B) EMPLACEMENT: remplace(6, "t")
   (ix) CARACTERISTIQUE ADDITIONELLE:
      (A) NOM/CLE: variation
      (B) EMPLACEMENT: remplace(9, "a")
   (ix) CARACTERISTIQUE ADDITIONELLE:
      (A) NOM/CLE: mutation
      (B) EMPLACEMENT: remplace (12, "g")
   (ix) CARACTERISTIQUE ADDITIONELLE:
      (A) NOM/CLE: mutation
      (B) EMPLACEMENT: remplace(12, "c")
   (ix) CARACTERISTIQUE ADDITIONELLE:
      (A) NOM/CLE: mutation
      (B) EMPLACEMENT: remplace (12, "t")
   (ix) CARACTERISTIQUE ADDITIONELLE:
      (A) NOM/CLE: mutation
      (B) EMPLACEMENT: remplace (15, "t")
   (ix) CARACTERISTIQUE ADDITIONELLE:
      (A) NOM/CLE: mutation
      (B) EMPLACEMENT: remplace(18, "a")
   (xi) DESCRIPTION DE LA SEQUENCE: SEQ ID NO: 6:
   TGGTGCGGTC CATGCAAGAT G 21
(2) INFORMATION POUR LA SEQ ID NO: 7:
   (i) CARACTERISTIQUES DE LA SEQUENCE:
      (A) LONGUEUR: 659 paires de bases
      (B) TYPE: acide nucléique
      (C) NOMBRE DE BRINS: deux
      (D) CONFIGURATION: linéaire
   (ii) TYPE DE MOLECULE: ADNC pour ARNm
   (iii) HYPOTHETIQUE: NON
   (vi) ORIGINE:
      (A) ORGANISME: Triticum aestivum
   (xi) DESCRIPTION DE LA SEQUENCE: SEQ ID NO: 7:
   CAAAGTGCGC GTGAGAAATA AGCGGTGCTT GCCCAGTAGA GAGAGAGAGA GAGAGAGAGA   60
   GAGATGGCGG CGTCGGCGGC GACGGCGACG GCGACGGCGG CGGCGGTAGG GGCGGGGGAG  120
   GTGATCTCCG TCCACAGCCT GGAGCAGTGG ACCATGCAGA TCGAGGAGGC CAACGCCGCC  180
   AAGAAGCTGG TGGTGATTGA CTTCACTGCA TCATGGTGCG GACCATGCCG CATTATGGCT  240
   CCAATTTTCG CTGATCTCGC CAAGAAGTTC CCAGCTGCTG TTTTCCTCAA GGTCGACGTT  300
   GATGAACTGA AGCCCATTGC TGAGCAATTC AGCGTGGAGG CCATGCCAAC CTTCCTGTTC  360
   ATGAAGGAAG GAGATGTCAA GGACAGGGTT GTCGGAGCTA TCAAGGAGGA ACTGACGACC  420
   AAGGTTGGCC TACACGCGGC CCAGTAATCA CCTACCGGAG TAGCATTCGC CTAAATAAAA  480
   TTGCCGCTCA ACAAGTAGTG CCTCTAATGG CACCTTATAT CCTGTGTACT GCTTGTTACT  540
   TGTTGGTTTA TGGATAATGG TGAATCAAGT GTGACTTTAT TCGGTAAATG GTTGATTTTC  600
   GTAAGGAGCT GATCGAATTC AGTTGTTCGG CTATAGGCAA AAAAAAAAAA AAAAAAAAA   659
(2) INFORMATION POUR LA SEQ ID NO: 8:
   (i) CARACTERISTIQUES DE LA SEQUENCE:
      (A) LONGUEUR: 630 paires de bases
      (B) TYPE: acide nucléique
      (C) NOMBRE DE BRINS: deux
      (D) CONFIGURATION: linéaire
   (ii) TYPE DE MOLECULE: ADNc pour ARNm
   (iii) HYPOTHETIQUE: NON
   (vi) ORIGINE:
      (A) ORGANISME: Triticum durum
   (xi) DESCRIPTION DE LA SEQUENCE: SEQ ID NO: 8:
   CGTGAGAAAT AAGCGGTGCT TGCCAAGCAG AGAGAGAGAG AGAGAGAGAG ATGGCGGCGG   60
   CGGCGACGGC GACGACTACA GCGGCGGCGA CGGCGGCGGC GGTGGGGCCG GGGGAGGTGA  120
   TCTCCGTCCA CAGCCTGGAG CAGTGGACCA TGCAGATCGA GGAGGCCAAC GCCGCCAAGA  180
   AGCTGGTGGT GATTGACTTC ACTGCATCAT GGTGCGGACC ATGCCGCATC ATGGCTCCAA  240
   TTTTTGCTGA TCTCGCCAAG AAGTTCCCAG CTGCTGTTTT CCTCAAGGTC GACGTTGATG  300
   AACTGAAGCC CATTGCTGAG CAATTCAGCG TCGAGGCCAT GCCAACCTTC CTGTTCATGA  360
   AGGAAGGAGA CGTCAAGGAC AGGGTTGTCG GAGCTATCAA GGAGGAGCTG ACGACCAAGG  420
   TTGGGCTCCA CGCGGCTGCC TAGTAATCAC CTAGCGGAGT AGTATTCGCC TAAATAAAAT  480
   TGCCGCTTGA GAAGTAGTGC CTCCAATGGC ACCGGATATG CTGTGTACTG CTTGCTTCTT  540
   GTGAGTTTAT GGATGATGGT GAATCAAGTG TGACTTTATT CGGTAAATGG TTGATTTCAT  600
   AAAAAAAAAA AAAAAAAAAA AAAAAAAAAA                                   630